# EUROPEAN PATENT SPECIFICATION

(11) **EP 2 730 297 B1**
(45) Date of publication and mention of the grant of the patent: **06.03.2019**
(21) Application number: 13191891.4
(22) Date of filing: 07.11.2013
(51) Int. Cl.: A61L 27/34, A61L 27/50, A61L 27/18

(54) **Graft fabric coating and methods**
Transplantattextilbeschichtung und -verfahren
Revêtement et procédés de tissu de greffe

(30) Priority: 09.11.2012 US 201261724629 P; 14.03.2013 US 201313828161
(43) Date of publication of application: 14.05.2014
(73) Proprietor: Cook Medical Technologies LLC, Bloomington, IN 47404 (US)
(72) Inventor: Wagner, Zachary, Noblesville, IN 46060 (US); Isch, Andrew, West Lafayette, IN 47906 (US)
(74) Representative: Williams Powell

(56) References cited:
- EP-A1- 0 651 005
- WO-A1-00/09041
- WO-A1-2012/166274
- WO-A1-2013/158586
- US-B1- 6 406 792
- Jamiel: "MED-6670 Low Coefficient of Friction Silicone Coating", , 17 October 2011 (2011-10-17), pages 1-4, XP055247445, Retrieved from the Internet: URL:https://www.silicone-polymers.co.uk/pd fMaster/MED-6670P.pdf [retrieved on 2016-02-04]

## Description

### TECHNICAL FIELD

Generally, the present invention relates to medical devices, such as vascular grafts. More particularly, the present invention relates to vascular grafts made of porous fabrics, such as polyethylene terephthalate (PET), having a unique silicone coating to improve resistance to permeability. Such a medical device may be useful in applications, such as endolumenal repair of an abdominal aortic aneurysm.

### BACKGROUND

Abdominal aortic aneurysm (AAA) is a leading cause of death in the United States, causing an estimated 15,000 mortalities each year. An abdominal aortic aneurysm is a bulge in the wall of the artery, usually a sequela of arteriosclerosis or the buildup of plaque on the inside of the artery. If untreated, the aneurysm may rupture, causing death.

One approach for the treatment of AAA involves invasive abdominal surgery. The abdomen is opened and the aneurysm is identified. The aorta is opened and a surgical graft is inserted into the aorta and sewn in place. The aorta is then closed over the graft.

More recently, stent grafts have been used in less-invasive procedures. Stent grafts include a graft layer inside and/or outside a stent structure. The stent graft provides a graft layer to reestablish a flow lumen through the aneurysm and a stent structure to support the graft and to resist occlusion or stenosis. Stent grafts may be inserted via incisions in the groin and deployed at the aneurysm site using a delivery catheter. Once in place, the stent graft expands within the aorta, providing a path for blood flow and reinforcing the weakened vessel.

A variety of materials have been used for vascular repair including polyethylene terephthalate (PET; Dacron^{(R)}; woven and knitted), Teflon™, bovine vessels, cryopreserved vessels of human or animal origin and others. Whether a traditional or stent graft, because of the size of the vessel being reinforced and the pressures within the vessel, AAA grafts must be made of strong and compliant textiles. Dacron®, for example, is an accepted and commonly used material for vascular repair, particularly for large diameter vascular grafts (>6 mm in diameter).

PET grafts are made in a similar fashion to most textiles. Fibers are woven or knitted into a specific geometry and structure. The result is a very strong "fabric" but one which is porous. The PET fibers facilitate the clotting mechanism of the body to seal the pores of the PET graft. The PET fabric itself is permeable to water and blood, however, once immersed in blood, the graft saturates, thrombosis and seals the graft. While this thrombosis works at preventing graft leakage, it is not consistent throughout the patient population. As such, because integrity against leakage of the graft is important, such vascular grafts are often preclotted to prevent leaking. Alternatively, graft pores have been sealed with collagen and other materials (e.g., Thoralon™). However, although collagen and other coatings may provide sealing to prevent initial blood losses, known coatings have not proven adequate for more long term needs (>1-3 months) or cases where fluid (e.g., serum or water) permeability is important. The permeability of grafts is a particular problem in PET grafts for endolumenal AAA repair. Because of the porous nature and insufficiency of currently available coatings, over time seepage develops between the PET grafts and the aorta. In the case of AAA, permeability of the graft to any fluid can lead to worsening of the aneurysm. As such a great need exists for grafts with decreased permeability due to reduced porosity without introducing substantial thickness or surface friction to the graft. Also, grafts that do not require natural thrombosis to seal the graft are highly desirable.

WO-2013/158586, WO 2012/166274, EP-0,651,005, US-6,406,792, WO-00/09041 and Journal "MED-6670 Low Coefficient of Friction Silicon Coating", www.silicone-plymers.co.uk, 17 October 2011 disclose silicone coatings for medical devices.

### SUMMARY

The scope of the invention is defined by the claims. Any references in the description to methods of treatment refer to the products of the present invention for use in a method for treatment.

In one embodiment, the invention relates to an implantable medical device comprising: (i) a polymeric layer of porous material having a first surface and a second surface, wherein the first surface and the second surface are positioned on opposite sides of the polymeric layer; and (ii) a non-porous silicone coating embedded within the polymeric layer and formed from a two-part dispersion of silicone elastomer material dispersed in xylene, the coating comprising silicone, ethyltriacetoxysilane, and xylene, disposed on at least the first surface of the polymeric layer to permeate the polymeric layer, wherein the polymeric layer or a portion thereof has a substantially reduced permeability or is entirely impermeable, wherein the embedded coating does not substantially increase the thickness of the implantable medical device, and wherein the embedded coating reduces the surface friction of the implantable medical device.

The non-porous coating may include additionally trace amounts of acetic acid. The polymeric layer may include a polyethylene terephthalate fabric, an ultra-high-molecular-weight polyethylene, or a polyurethane material comprising a soft segment and a hard segment. The device may be a vascular graft, a stent graft, or a vascular patch. The device may further include a reinforcing layer disposed on an outer most surface of the polymeric layer and non-porous silicone coating that includes a plurality of stents. The stents may include a material selected from stainless steel, nickel, silver, platinum, palladium, gold, titanium, tantalum, iridium, tungsten, a self-expanding nickel titanium alloy, nitinol, and Inconel.

In another embodiment, the invention relates to a method of making a polyethylene terephthalate (PET) graft having a reduced permeability, comprising: providing a porous graft having a first surface and a second surface, wherein the first surface and the second surface are positioned on opposite sides of the polymeric layer; and applying a silicone composition comprising a two-part dispersion of silicone elastomer material dispersed in xylene to at least the first surface of the graft, said silicone composition permeating the graft so as to become embedded in the graft to produce a pore-free coating on the graft, the coating comprising silicone, ethyltriacetoxysilane and xylene, such that the graft or a portion thereof has a substantially reduced permeability or is entirely impermeable, wherein the coating does not substantially increase the thickness of the graft, and wherein the coating reduces the surface friction of the graft.

The method may further include a step of pretreating the graft comprising washing the graft in methylene chloride, acetone, or a mixture thereof. The method may include curing the coated graft with an increased temperature in a range from about 30°C to about 150°C following the applying step.

In yet another embodiment, the invention relates to a method for making a vascular prosthesis comprising providing a porous graft layer comprising a porous PET fabric, the graft layer having a first surface and a second surface, wherein the first surface and the second surface are positioned on opposite sides of the polymeric layer; and applying a silicone composition comprising a two-part dispersion of silicone elastomer material dispersed in xylene to at least the first surface of the graft, said silicone composition permeating the graft layer so as to become embedded in the graft to produce a first pore-free coating on the graft layer, the coating comprising silicone, ethyltriacetoxysilane, and xylene, such that the graft layer or a portion thereof has a substantially reduced permeability or is entirely impermeable, wherein the coating does not substantially increase the thickness of the graft layer, and wherein the coating reduces the surface friction of the graft layer. The method may further include applying the silicone composition to the second surface. In the method, the step of applying the silicone composition may include applying the composition by spraying the silicone composition on the first surface of the graft layer. The method may further include curing the silicone material with an increased temperature in a range from about 30°C to about 150°C.

A method of repairing or treating a defective vessel in an individual is described. The method includes reinforcing or replacing the defective vessel with an implantable medical device that includes a polymeric layer having a first surface and a second surface, wherein the first surface and the second surface are positioned on opposite sides of the polymeric layer and a first non-porous silicone coating that includes a two-part dispersion of silicone material in an organic solvent. The silicone coating is disposed on at least the first surface of the polymeric layer, such that the at least first surface of the polymeric layer or a portion thereof has a substantially reduced permeability or is entirely impermeable. The coating does not substantially increase the thickness of the implantable medical device and the coating reduces the surface friction of the implantable medical device.

Also described is a method of treating aortic aneurysm comprising implanting into a patient an implantable medical device as defined in the claims.

Also described is a method of treating thoracic aneurysm comprising implanting into a patient an implantable medical device as defined in the claims.

### BRIEF DESCRIPTION OF THE DRAWINGS

Embodiments of the present invention are described below, by way of example only, with reference to the accompanying drawings, in which:
Figure 1 depicts an exemplary coated stent graft of the present invention;
Figures 2A-B depict results from a 60 second water drop test;
Figures 3A-B depict results from a 4 minute water drop test;
Figures 4A-B depict puncture holes in test LP grafts; and
Figures 5A-B depict effects of collateral leakage through the puncture holes shown in Figures 4A-B, respectively.

The components in the drawings are not necessarily to scale, emphasis instead being placed upon illustrating the principles of the teachings herein.

### DETAILED DESCRIPTION OF THE DRAWINGS AND THE PRESENTLY PREFERRED EMBODIMENTS

The present invention is directed to medical devices, including vascular grafts made of porous fabrics, such as Dacron®, also known under its generic name as polyethylene terephthalate (PET), treated with a silicone composition including a two-part dispersion of silicone elastomer in an organic solvent to prevent leakage of fluid through the pores of the graft and, also, to seal any sutures, seams and other intentional punctures in the graft. Specifically, the present invention uses blood-compatible silicone compositions, MED-6670 and MED10-6670 commercially available from NuSil Technology LLC, Carpinteria, CA that include a two-part silicone elastomer material dispersed in xylene, as a coating for at least the blood-contacting surface of the graft.

The terms "coated" and "coating" refer to the application of a coating material to a portion of or the entire surface of a medical device by coating, dipping, spraying or other suitable method of application of coatings to medical devices.

The terms "silicone-coated," "a silicone coating," and "a coating" refer to the application of a silicone composition comprising a two part dispersion of silicone material in an organic solvent, such as xylene to a portion of or the entire surface of a medical device.

The silicone compositions of the present invention include NuSil's MED-6670 and MED10-6670. MED-6670 and MED10-6670 are approved for implant applications and offer thromboresitance, high tensile strength, and superb flexibility. MED-6670 and MED10-6670 are biostable and useful *in vivo* in long term blood containing applications requiring biostability and leak resistance for a period extending one year or more. MED-6670 is an unrestricted form that may be considered for long-term implant applications (i.e., ≥30 days); MED10-6670 is a restricted form that may be considered for short-term implant applications (i.e., ≤29 days) or external devices. MED-6670 and MED10-6670 include the same components, but the MED-6670 composition is processed through additional good manufacturing practice (GMP) processes to be considered "unrestricted."

MED-6670 and MED10-6670 silicone compositions are sold as a two-part (Part A and Part B) silicone elastomer dispersed in xylene. The term "a two-part dispersion" means that the components of the composition are not fully dissolved in the solvent. It's not fully a solution, but not necessarily a suspension. Part A includes a silicone crosslinker (ethyltriacetoxysilane) at about 62%, xylene at about 17%, and trace amounts of acetic acid (which is given off during cure). It is believed that ethyltriacetoxysilane undergoes hydrolysis to create acetic acid and a Trisilanol, which then polymerizes into low molecular weight siloxane oligomers. Part B includes 70% xylene. Parts A and B are used at a Mix Ratio of 1:1 (Part A : Part B), as per manufacturer's instructions (see NuSil Technology LLC, Carpinteria, CA).

A thin coat of MED-6670 or MED10-6670 cures rapidly (i.e., within minutes) with elevated temperatures. Once cured, the coating is chemically bonded to the graft material and mimics the mechanical properties of the graft material. The result is a durable yet flexible coating that resists abrasion from moving, sliding and rubbing parts. It achieves this with a smooth finish that also results in as much as 50% decrease in coefficient of friction when coated graft samples vs. non-coated graft samples are compared side by side.

Although the invention is described herein in the context of fabric grafts, the silicone coating may also be applied to other porous graft materials, such as ePTFE (expanded polytetrafluoroethylene) or polyurethanes, such as Thoralon™ to prevent leakage of fluid through the pores of the graft to seal any sutures, seams and other intentional punctures in the graft.

Moreover, although the invention is described herein in the context of vascular grafts, the silicone coating may also be applied to other medical devices such as stent grafts, synthetic heart valve, hernia mesh or other permeable matrix-like devices where porosity/sealing may require modifications.

Primary indications for the silicone-coated medical devices of the present invention are AAA, thoracic aneurysms, venous grafts, and stenotic heart valves.

In general, silicone possesses a number of desirable properties, such as blood compatibility, biocompatibility and biostability, compliance, and strength, which are important in many vascular applications. In addition, MED-6670 and MED10-6670 silicone compositions provide further advantages.

First, the unique property of MED-6670 and MED10-6670 compositions used to coat medical devices of the present invention is that these compositions have much lower coefficient of friction than most other silicone materials available on the market and are able to reduce the coefficient of friction of the graft fabric by making it smoother. Because of the much lower coefficient of friction of the MED-6670 and MED10-6670 compositions, the resultant MED-6670- and MED10-6670-coated grafts have at least 1%, more preferably at least 5%, more preferably at least 10%, more preferably at least 15%, more preferably at least 20%, more preferably at least 25%, and most preferably at least 50% or more decrease in coefficient of friction when coated graft samples vs. non-coated graft samples are compared side by side. The surface friction of the MED-6670- and MED10-6670-coated devices is therefore reduced.

Second, the MED-6670 and MED10-6670 coatings adhere to the graft, seal the pore openings, create a barrier against blood perfusion, and maintain their mechanical function (e.g., prevent seepage between the graft and artery) *in vivo* for a period of years. As such, MED-6670 and MED10-6670-coated textiles according to the present invention have improved impermeability (i.e., are less prone to allow leakage of fluids, such as serum or water, through the body of the graft, both long and short term). Specifically, the permeability of the silicone-coated PET graft is preferably from 0 to 100 mL/cm²/min; preferably, the permeability of the silicone-coated graft is preferably from 0 to 50 mL/cm²/min; more preferably, the permeability of the silicone-coated graft is from 0 to 25 mL/cm²/min; most preferably, the permeability of the silicone-coated graft is from 0 to 10 mL/cm²/min or even less, e.g., from 0 to 5 mL/cm²/min.

By using the MED-6670 and MED10-6670 coatings the pores of a porous PET stent graft are effectively sealed, thereby reducing the permeability of a PET graft without altering the profile of the material by more than 50%; more preferably by 75%; more preferably by 80%; more preferably by 85%; more preferably by 90%; more preferably by 95%; more preferably by 98%; more preferably by 99%; and most preferably by 100%. The pores of a porous PET stent graft are effectively sealed, thereby reducing the permeability of a PET graft without altering the profile of the material by at least 50% or more.

Importantly, the silicone-coated grafts of the present invention have improved impermeability to blood without relying on natural clotting mechanism. Specifically, a thin silicone material once infused into graft material creates a barrier against blood profusion, increasing structural integrity of the graft fabric material without adding bulk or thickness, and reducing the coefficient of friction of the graft by making it smoother.

Next, as mentioned immediately above, the silicone-coated grafts of the present invention have essentially the same thickness as the untreated material and reduced surface friction as compared to untreated graft materials (i.e., the thickness of the graft material was unchanged after applying the MED-10-6610 material, both measuring 0.0040"). Ideally, the profile of a graft should be thin to allow for the smallest possible endolumenal intervention. NuSil MED-6670 biomaterial has been successfully applied such that the coating is basically wicked into the graft fibers and seals the fibers together when it is cured (the change in thickness of the material could not be measured with a standard micrometer, i.e. the silicone coating does not substantially change the thickness as compared to the un-coated PET graft). Also, these MED-6670 and MED10-6670 silicone coatings have a relatively low viscosity which allows for spraying or dipping of graft material, the organic solvent (xylene) allows for wetting of the fabric. This in turn allows the silicone to work into and between individual fibers of the graft fabric in a very short amount of time, i.e., within seconds and less than 1 minute. As the organic solvent quickly evaporates, the volume of material applied to the fabric diminishes, and a thin embedded layer of silicone is left within. The final product is essentially as thin as before the coating was applied (i.e., the coating has no measurable thickness); the material is virtually impermeable to liquids and the coefficient of friction is equal or less than the coefficient of friction of an untreated graft fabric.

Also, the silicone coating strengthens the fabric and prevents it from fraying or ripping. The fabric becomes a composite as the coating wicks the silicone into the weave, which in turn adds strength. As such, silicone-coated textiles according to the invention provide strong and compliant reinforcement or replacement of the diseased area.

Accordingly, grafts coated according to the invention may be used in a variety of applications, including vascular grafts, stent grafts and vascular patches. Grafts according to the invention are particularly useful in the repair of AAA. Also, as mentioned above, the coating seals sutures, seams and other intentional punctures of the graft.

Moreover, the MED-6670 and MED10-6670 silicone coatings not only provide a nonthrombogenic and an improved blood compatible lumen surface, but may also be used as a drug delivery vehicle (e.g., deliver a pharmacological agent) and as a surface-modifying coating to alter mechanical properties such as compliance and wear resistance.

In view of this, the coatings of the present invention are particularly useful for coating Dacron™ to create a graft having a very thin profile yet having improved resistance to permeability, which is highly beneficial in endovascular applications.

As such, the present invention solves the problem of seepage between the graft and blood vessels, such as aorta through the pores of the fabric occurring with currently commercially available coated Dacron™ grafts. In addition to PET, grafts or patches made of other porous materials, such as ePTFE, may be effectively sealed.

For example, in an embodiment where a heart valve is coated with the silicone composition of the present invention, the silicone material provides synthetic valve leaflets with the ability to seal and resist clotting, which may otherwise compromise the valve. Also, the silicone coating aids in shape retention and sealing properties as the leaflets close.

In another example, where Thoralon™ graft material may be coated with the silicone composition, the low coefficient of friction silicone allows the graft to be deployed easily, as the high friction Thoralon™ can cause higher deployment forces. The Thoralon™ structure first requires a thin base of Thoralon™ to be applied to the graft, then a final layer of the low coefficient of friction silicone may be applied to the surface of the device.

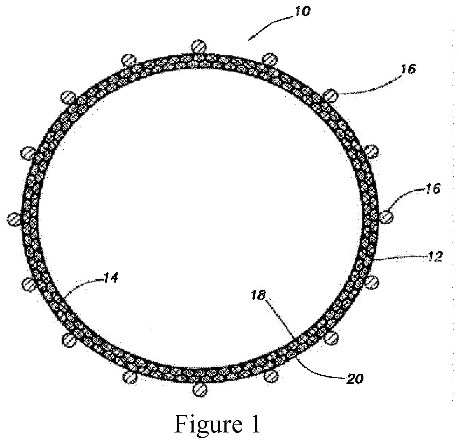

The silicone coating of the present invention allows grafts, valves or any other coated devices to be impermeable to blood without relying on natural clotting mechanism. Specifically, a thin silicone material is infused into graft material to create a barrier against blood profusion, increasing structural integrity of the graft fabric material without adding bulk or thickness, and reducing the coefficient of friction of the graft by making it smoother. FIG. 1 depicts a preferred embodiment of an AAA stent graft according to the present invention. Referring to FIG. 1, AAA stent graft **10** comprises a cylindrical PET fabric **12**. The cylindrical PET fabric **12** has a first surface **18** and a second surface **20**. Typically, the first surface **18** is the inner device surface which contacts the blood and the second surface is the outer surface, which contact the tissue/artery. A silicone coating **14** is disposed on at least the first surface **18** (i.e., the inner surface) and, optionally, on the second surface **20** (i.e., the outer surface).

In certain embodiments, as seen in FIG. 1, the silicone composition preferably permeates or infiltrates the spaces between the woven fibers of fabric graft material.

In certain embodiments a reinforcing layer **16** may be disposed on the outer most surface of the graft material and the coating. Reinforcing layer may comprise a plurality of stents made of stainless steel, nickel-titanium alloy, silver, platinum, palladium, gold, titanium, tantalum, iridium, tungsten, a self-expanding nickel titanium alloy, nitinol, and Inconel or another suitable material. Alternatively or additionally, the reinforcing layer may be disposed on the inner most surface of the graft material and the coating.

Accordingly, one embodiment of the invention is directed to a vascular graft comprising a graft layer made of a PET fabric. The graft layer is preferably configured or shaped for use in a vessel or for repair of a vessel having an internal diameter of more than 2 mm. More preferably, the vessel has an internal diameter of more than 3 mm, and, most preferably, more than 6 mm. The graft layer has a first surface and a second surface, where the first surface and the second surface are positioned on opposite sides of the graft layer. Preferably, a non-porous silicone coating is disposed on at least the first surface of the vascular graft.

This coating comprises a two-part dispersion of silicone in organic solvent (xylene), such as MED-6670. The coated first surface is preferably the blood interface surface of the graft. Alternatively or in addition, a non-porous silicone coating is disposed on the second surface the graft, which is the artery/tissue interface surface.

In other words, the coating may be applied to either surface of the graft. In yet another embodiment, the coating may be applied to both surfaces of the graft.

In another embodiment a first coating applied to the first surface may permeate through and form a cover or a second coating on the surface positioned on the opposite side of the graft. For example, the silicone coating applied to the first surface to form the first coating may penetrate the polymeric graft layer and form the second coating on the second surface. In another embodiment, a second coating may be applied to the opposite (second or tissue-contacting) surface of the graft.

The graft layer may have any desired shape, such as a cylinder, a bifurcated/Y-shaped cylinder, or a substantially flat sheet for patches.

In certain embodiments, at least a portion of the graft layer has a substantially cylindrical shape and is a graft having an internal diameter of greater than 2 mm, and, more preferably, greater than 3 mm. Even more preferably, the graft is a large bore graft, in which the internal diameter is greater than 6 mm and the outer diameter is greater than 6.1 mm. For example, the external diameter may be designed or configured to fit in a large bore vessel, such as an abdominal aorta of an adult human. Alternatively or additionally, it may be designed to fit within other vessels, such as a human femoral artery or carotid artery.

The first surface is preferably disposed on the inner surface of the cylindrical graft layer, i.e., the first coating is on the first surface of the cylinder. The second surface is disposed on the outer surface of the cylindrical graft layer, i.e., the second coating may be on the second surface of the cylindrical graft layer. If desired, the graft may have a coating on both surfaces, i.e., both the first and second surfaces. The coatings may be applied separately to each side or, as noted, when the first coating is applied to one surface, it may permeate through and form a second coating on the opposing surface of the graft.

The coating on the PET fabric comprises a silicone composition as defined in the claims, such as MED-6670 or MED10-6670. In another embodiment, the present invention relates to a composite graft that includes a polymeric layer having a first surface and a second surface, where the first surface and the second surface are positioned on opposite sides of the polymeric layer, where the first surface is the blood-contacting surface and the second surface is the tissue/artery-contacting surface; and a first non-porous silicone coating comprising a two-part dispersion of silicone material in an organic solvent, the silicone coating disposed on at least the first surface of the polymeric layer such that at least the first surface of the polymeric layer has a substantially reduced permeability or is impermeable (as compared to the graft having an uncoated first surface), where the coating does not substantially increase the thickness of the medical device, and where the coating reduces the surface friction of the medical device (as compared to the uncoated graft).

Although the present invention provides for a biocompatible, leak-resistant graft using a single coating material, if desired, other coating(s) may also be applied in conjunction with the silicone coating of the present invention.

For example, in certain embodiments, an additional non-silicone coating may be applied to the graft. The additional non-silicone coating may be applied before or after the silicone coating is applied to at least one of the surfaces of the graft.

The additional coating may include THORALON™ biomaterial. The THORALON™ coating may comprise one or more polyurethanes, or mixtures and combinations thereof. Preferably, the polyurethanes each comprise a soft segment and a hard segment. The soft segment may comprise one or mere compounds selected from the group consisting of polytetramethylene oxide, polyethylene oxide, polypropylene oxide, polycarbonate, polyolefin, polysiloxane (e.g., polydimethylsiloxane), polyether soft segments made from higher homologous series of diols, and mixtures and combinations thereof. The soft segments may also have either alcohol or amine end groups. The hard segment is comprised of an isocyanate (preferably a diisocyanate) and an amine (preferably a diamine) or a polyol.

In another embodiment, the hard segment may comprise an isocyanate and both an amine and a polyol. The isocyanate component of the hard segment may comprise one or more compounds selected from the group consisting of 4,4'-diphenylmethane diisocyanate (MDI), tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl2,4-diisocyanate, 4,4'-biphenylene diisocyanate, 3,3-dimethoxy-4,4'-biphenyl diisocyanate, and mixtures and combinations thereof. The amine component of the hard segment may comprise one or more compounds selected from the group consisting of ethylene diamine, propane diamines, butanediamines, hexanediamines, pentane diamines, heptane diamines, octane diamines, m-xylylene diamine, 1,4-cyclohexane diamine, 2-methypentamethylene diamine, 4,4'-methylene dianiline, alkanol amines and diamines, and mixtures and combinations thereof. The polyol component of the hard segment may comprise one or more compounds selected from the group consisting of ethylene glycol, diethylene glycol, triethylene glycol, 1,4-butanediol, neopentyl alcohol, 1,6-hexanediol, 1,8-octanediol, propylene glycols, 2,3-butylene glycol, dipropylene glycol, dibutylene glycol, glycerol, and mixtures and combinations thereof.

In certain embodiments, the present invention relates to medical devices, such as vascular grafts made of other porous polymeric materials, such as Thoralon™ and coated with a silicone as defined in the claims, such as MED-6670 or MED 10-6670 to prevent leakage of fluid through the pores of the Thoralon™ graft. Specifically, the present invention uses blood-compatible silicone material as defined in the claims, as a coating for the blood-contacting graft materials.

Since MED-6670 and MED 10-6670 silicone compositions are dispersed in xylene and because the base material of Thoralon™, polyurethane is also dissolvable in xylene, the two materials may be bonded together during the dipping or spraying processes. This allows the Thoralon™ graft to have a very low coefficient of friction on the surface while retaining the mechanical properties of polyurethane.

In an embodiment where porous Thoralon™ graft is coated with the silicone composition, the low coefficient of friction silicone allows the graft to be deployed easier as compared to the uncoated Thoralon™ graft. Otherwise, the high friction Thoralon™ can cause higher deployment forces.

In certain embodiments, the invention is also directed to methods of making composite grafts having reduced permeability, including the PET composite grafts.

As discussed previously, the coating (e.g., in slurry or solution form) may be applied in a variety of ways, including but not limited to, spraying, dipping, applying with rollers or brushes, or casting. These processes can be applied on a textile sheet, graft or final product construct (e.g., stented graft) to improve the functional performance of the product by reducing permeability. As noted, the coating material applied to one side may permeate through and form a cover or a coating on both surfaces of the graft.

As noted, the actual application of the coating of the invention may be accomplished in a number of ways. For example, the silicone composition may be applied using a brush, followed by heating the coated part in an oven while rotating the coated part to drive off the solvent. One or more coating layers may be applied, if desirable. However, in preferred embodiments of the present invention, the final product is a composite graft fabric that is essentially as thin as graft fabric before the application of the silicone coating of the present invention.

In another embodiment, the silicone composition may be sprayed onto the fabric using a spray nozzle and the coating may be dried. In another embodiment, the graft fabric may be dip coated. Useful methods for dip coating and spray coating are described, for example, in U.S. Pat. No. 5,104,400 to Berguer et al.

In a preferred method, the coating applied to the PET graft is formed from a solution of a two-part dispersion of silicone as defined in the claims, such as NuSil MED-6670 that readily penetrates the graft. By using such solutions, no pressure is needed to get the coating to permeate into the pores of the graft. The unique property of this silicone composition is that it has a much lower coefficient of friction than most other silicone materials available. This material also has a relatively low viscosity which allows for spraying or dipping of graft material, and the organic solvent allows for wetting of the graft fabric. This allows the silicone to wick into and between the individual fibers of the graft material in a very short amount of time. As the organic solvent quickly evaporates, the volume of material applied to the fabric diminishes and a thin embedded layer of silicone is left within. The fabric becomes a composite as it wicks the silicone into the weave. Preferably, the silicone is then quickly cured in place with thermal energy (heat) or high-energy light sources, such as UV. The final product is a composite graft fabric that is essentially as thin as before the coating was applied, impermeable to liquids and the coefficient of friction is equal or less than uncoated graft fabric.

In another embodiment, the present invention is directed to a method for making a vascular prosthesis comprising providing a graft layer comprising a porous PET fabric, the graft layer having a first surface and a second surface, where the first surface and the second surface are positioned on opposite sides of the graft layer; and coating at least the first surface of the graft layer (i.e., the blood contacting surface) with a silicone composition (e.g., in slurry or solution form) to produce a first pore-free coat on the first surface. The graft layer is preferably configured for use in a vessel or to repair a vessel having an internal diameter of more than 2 mm. More preferably, the vessel has an internal diameter of more than 3 mm, and, most preferably, more than 6 mm. The silicone composition may penetrate the graft layer to produce a second coat on the second surface. In another embodiment, the second surface may be separately coated with the silicone composition of the present invention. The silicone composition preferably comprises a two-part dispersion of silicone as defined in the claims, such as MED-6670 or MED10-6670, as described herein.

The step of coating may be accomplished by spraying at least one layer of the silicone composition on the first surface of the graft layer and allowing the layer to dry. In another embodiment, the solvent is evaporated and the silicone material quickly cured with elevated temperature. As the organic solvent quickly evaporates, the volume of composition applied to the graft fabric diminishes, and a thin embedded layer of silicone is left within the graft fabric.

These steps may be repeated one or more times if additional coating layers of the silicone compositions are desired and/or until the desired thickness is obtained. However, if no measurable thickness of the silicone coating is desired, only an amount of silicone sufficient to seal the pore openings of the graft material is provided.

The step of coating may include applying the silicone composition to the first surface with a brush or roller. In this embodiment, the method may further comprise heating the coated surface to drive off any solvent, as described above. In another embodiment, the step of coating may include dipping the graft fabric in a slurry of the silicone composition one or more times.

Regardless of the application process, drying of the coating is optimally accomplished at temperatures in a range of about 30°C to about 150°C, and most preferably, at a temperature about 150°C.

In another variation, the step of coating may include coating at least one of the first surface and the second surface with a silicone monomer and polymerizing or curing the monomer to form the polymer composition by thermal energy or high energy light.

Optionally, the method may further comprise pretreating the graft layer by washing in methylene chloride, acetone, or another suitable agent to enhance adhesion of the silicone to the textile. If desired, one or more additives may be added to the silicone composition to promote effective bonding of the silicone composition to the graft layer.

In another embodiment, the invention relates to a method for sealing the pores of a porous PET graft to produce a composite graft with a substantially reduced permeability or PET graft that is entirely impermeable.

The method comprises the steps of providing the porous PET graft having a first surface and a second surface, where the first surface and the second surface are positioned on opposite sides of the PET graft, and applying a silicone composition to at least the first surface of the PET graft (i.e., the blood contacting surface of the graft) to produce a pore-free coat on the surface of the PET graft composite. The silicone composition comprises a two-part dispersion of silicone as defined in the claims, such as MED-6670 or MED10-6670. The coating may be applied to the graft by dipping a part or the entire graft in the silicone composition or spraying the solution over the graft or by any other suitable method of coating and as per manufacturer's instructions. The solvent is then quickly evaporated from the graft material by holding the graft for at least 5 minutes at ambient temperature and humidity in a vented environment (fume hood), and cured by exposing the graft for 5 minutes to a temperature of 150°C. However, the materials may cure at lower temperatures, but the curing process will take longer. For example, the graft material may be cured by exposing the graft to 70°C for 20 minutes.

As such, preferably, the graft material is cured at temperatures in a range from about 30°C to about 150°C, and most preferably, at a temperature of about 150°C to produce a composite PET graft composite having a substantially reduced permeability or entirely impermeable, where the coating does not substantially increase the thickness of the medical device, and where the coating reduces the surface friction of the medical device.

The composite graft is preferably configured for use in a vessel or to repair a vessel having an internal diameter of more than 2 mm. More preferably, the vessel has an internal diameter of more than 3 mm, and, most preferably, more than 6 mm. Preferably, the composite graft comprises an AAA stent graft and the silicone composition comprising a two-part dispersion of silicone as defined in the claims, such as NuSil MED-6670.

The graft may be used in a variety of applications, including as a vascular graft, as a stent graft, or as a vascular patch. The graft is particularly useful for large bore vessels having an internal diameter of 6 mm or more. In a preferred embodiment, the graft comprises an AAA stent graft, such as Zenith Flex® endovascular graft supplied by COOK Inc., Bloomington, IN. Other suitable grafts may also be used. In this embodiment, the stent graft may further comprise a more rigid component, such as a stent or other suitable component to provide structural support to the stent graft. Preferably, the reinforcement is made of stainless steel or nickel-titanium alloy.

As such, in certain embodiments, the present invention relates to a method of treating AAA including implanting a vascular device comprising a composite graft of the present invention. The composite graft includes a polymeric layer having a first surface and a second surface, where the first surface and the second surface are positioned on opposite sides of the polymeric layer, where the first surface is the blood-contacting surface and the second surface is the tissue/artery-contacting surface; and a first non-porous silicone coating comprising a two-part dispersion of silicone material in an organic solvent, the silicone coating disposed on at least the first surface of the polymeric layer such that the at least the first surface of the polymeric layer has a substantially reduced permeability or is entirely impermeable, where the coating does not substantially increase the thickness of the medical device, and where the coating reduces the surface friction of the medical device.

In additional embodiments, the present invention relates to a method of repairing or treating a defective vessel in an individual. The method includes reinforcing or replacing the defective vessel with a composite graft of the present invention. The composite graft includes a polymeric layer having a first surface and a second surface, where the first surface and the second surface are positioned on opposite sides of the polymeric layer, where the first surface is the blood interface surface and the second surface is the tissue/artery contacting surface; and a first non-porous silicone coating comprising a two-part dispersion of silicone material in an organic solvent, the silicone coating disposed on at least the first surface of the polymeric layer such that the at least first surface of the polymeric layer has a substantially reduced permeability or is entirely impermeable, where the coating does not substantially increase the thickness of the medical device, and where the coating reduces the surface friction of the medical device. The individual may be any animal, and is preferably a mammal, such as a human. The defective vessel may be any vessel, and is preferably a large bore vessel, such as the abdominal aorta of a human.

Additionally, the silicone coating may be used with a variety of other medical devices including a graft, a stent graft, synthetic heart valve, hernia mesh, or other permeable matrix-like devices where porosity/sealing requires modification. The silicone-coated devices may be utilized by medical professionals (i.e., cardiologists, cardio-thoracic surgeons, etc.). Preferably, the coated medical devices of the present invention are for use in the treatment or repair of defective vessels, treatment of AAA or thoracic aneurysm, but also used as venous grafts, vascular patches, or stenotic heart valves.

The following examples are offered to illustrate embodiments of the invention, and should not be viewed as limiting the scope of the invention.

### EXAMPLES

### Example 1 - Water Permeability Bench Testing (60-Second Water Drop Test)

Permeability of an AAA-LP graft material coated with MED10-6670 was tested in comparison with the permeability of an untreated (i.e., uncoated) AAA-LP graft material. To produce the MED10-6670 coated AAA-LP graft, a solution of MED10-6670 was prepared per manufacturer instructions (1:1 ratio mixture of Part A and Part B http://www.nusil.com/library/products/MED10-6670P.pdf; NuSil Silicone Technology, Carpinteria, CA). The graft to be challenged was submersed into the MED 10-6670 for 60 seconds and then slowly removed as to not create droplets of solution at the distal end of the graft. The graft was suspended in a fume hood for approximately 15 minutes to ensure solvent evaporation and then placed in a 70°C convection oven for 20 minutes. The material was fully cured at this time. The graft was then subjected to Water Permeability Bench Testing.

To test the permeability of the coated and uncoated AAA-LP graft materials, a 60 second water drop test was performed with the AAA-LP graft material coated with MED10-6670 and the uncoated AAA-LP graft material.

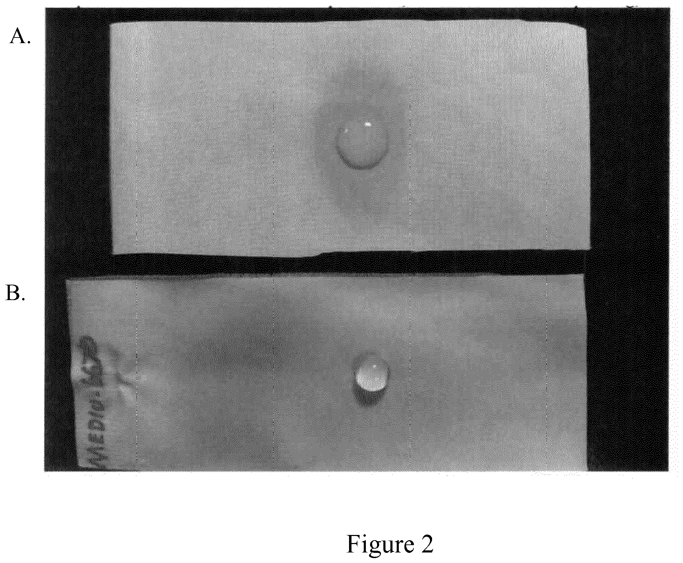

As seen in FIGS. 2A-B, the application of a drop of water to the untreated graft for 60 seconds resulted in water leaking through the graft (A). In contrast, application of a drop of water to the MED10-6670-coated graft for the same amount of time did not result in water leakage through the graft (B). Rather, the water droplet was "sitting" on the graft without showing any signs of absorption into the graft or leakage.

### Example 2 - Water Permeability Bench Testing (4-Minute Water Drop Test)

Permeability of an AAA-LP graft coated with MED10-6670 was tested in comparison with the permeability of an untreated (i.e., uncoated) AAA-LP graft.

The silicone-coated AAA-LP graft material was prepared as described in Example 1 above. To test the permeability of the coated and uncoated AAA-LP grafts, a 4 minute water drop test was performed with the AAA-LP graft treated with MED10-6670 and the untreated AAA-LP graft.

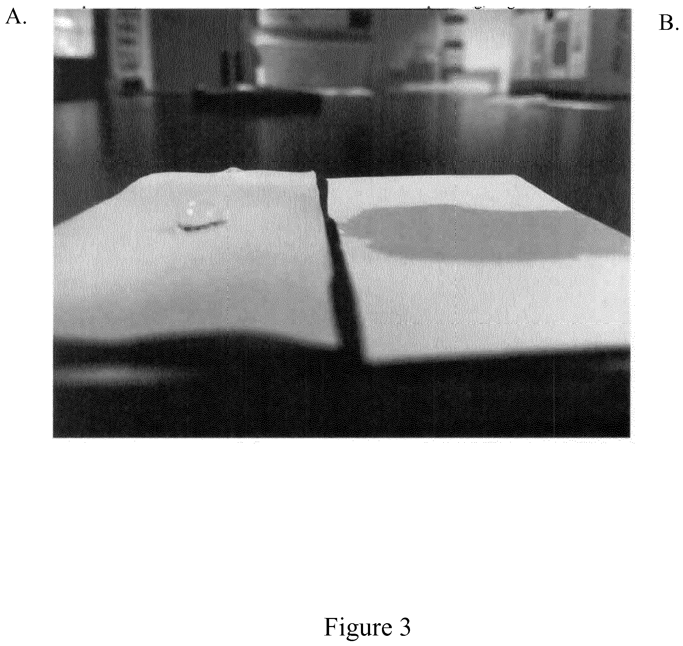

As seen in FIGS. 3A-B, the application of a drop of water to the untreated graft for 4 minutes resulted in water being absorbed into the graft and leakage through the graft (B). In contrast, application of a drop of water to the MED10-6670-coated graft for 4 minutes did not result in water leakage through the graft (A). Rather, the water droplet was "sitting" on the graft without showing any signs of absorption into the graft or leakage.

### Example 3 - Effect of Collateral Leakage

Permeability of an AAA-LP graft coated with MED-6670 was tested in comparison with the permeability of an untreated (i.e., uncoated) AAA-LP graft.

The silicone-coated AAA-LP graft was prepared as described in Example 1 above.

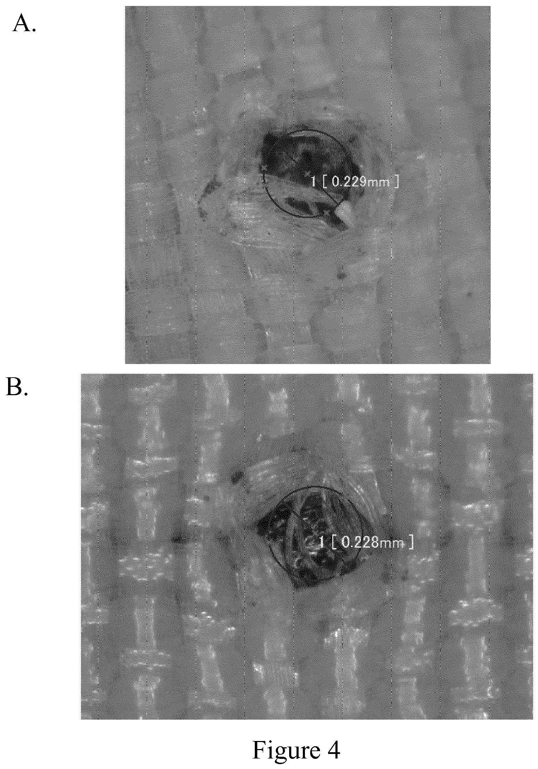

To test the permeability of the coated and uncoated AAA-LP grafts, both pieces of graft material were first punctured with Prolene 3-0 suture. The puncture sizes were 0.229 mm for the coated LP graft and 0.228 mm for the untreated graft. Figure 4 illustrates the punctures in the coated (A) and untreated (B) LP graft materials.

The punctured graft materials were then tested at 120 mmHg water pressure. Specifically, the graft sections to be tested were placed on a plate with a hole in it, and a pressure head of 120mm Hg is placed on top of the grafts using a cylinder of water. The amount of water leakage over time is then measured.

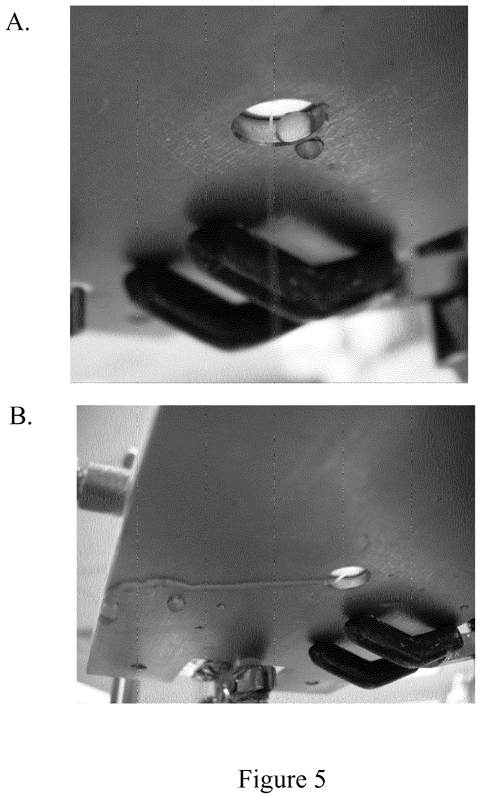

As seen in FIGS. 5A-B, permeability through the suture of the MED-6670-coated LP graft was about 3.8 mL/cm²/min as compared to the significantly higher permeability of the uncoated LP graft, which demonstrated 35.4 mL/cm²/min permeability. In other words, the leakage through the puncture of the uncoated LP graft was significantly greater as compared to the minimal leakage through the puncture in the MED-6670-coated LP graft.

## Claims

1. An implantable medical device comprising:
(i) a polymeric layer of porous material having a first surface and a second surface, wherein the first surface and the second surface are positioned on opposite sides of the polymeric layer; and
(ii) a non-porous silicone coating embedded within the polymeric layer and formed from a two-part dispersion of silicone elastomer material dispersed in xylene, the coating comprising silicone, ethyltriacetoxysilane, and xylene, disposed on at least the first surface of the polymeric layer to permeate the polymeric layer,
wherein the polymeric layer or a portion thereof has a substantially reduced permeability or is entirely impermeable,
wherein the embedded coating does not substantially increase the thickness of the implantable medical device, and
wherein the embedded coating reduces the surface friction of the implantable medical device.

2. The device of claim 1, wherein the embedded non-porous coating comprises silicone, ethyltriacetoxysilane and xylene.

3. The device of any preceding claim, wherein the polymeric layer includes at least one of a polyethylene terephthalate fabric; an ultra-high-molecular-weight polyethylene; and a polyurethane material comprising a soft segment and a hard segment.

4. The device of any of claims 1 and 2, wherein the polymeric layer comprises a polyurethane material comprising a soft segment and a hard segment, wherein the soft segment comprises one or more compounds selected from the group consisting of polyethylene oxide, polypropylene oxide, polytetramethylene oxide, polycarbonate, polyolefin, polysiloxane, polyether soft segments made from higher homologous series of diols, and mixtures and combinations thereof.

5. The device of any of claims 1 and 2, the polymeric layer comprises a polyurethane material comprising a soft segment and a hard segment wherein the hard segment comprises one or more compounds selected from the group consisting of 4,4'-diphenylmethane diisocyanate, tetramethylene diisocyanate, hexamethylene diisocyanate, trimethyhexamethylene diisocyanate, tetramethylxylylene diisocyanate, 4,4'-decyclohexylmethane diisocyanate, dimer acid diisocyanate, isophorone diisocyanate, metaxylene diisocyanate, diethylbenzene diisocyanate, decamethylene 1,10 diisocyanate, cyclohexylene 1,2-diisocyanate, 2,4-toluene diisocyanate, 2,6-toluene diisocyanate, xylene diisocyanate, m-phenylene diisocyanate, hexahydrotolylene diisocyanate (and isomers), naphthylene-1,5-diisocyanate, 1-methoxyphenyl2,4-diisocyanate, 4,4'-biphenylene diisocyanate, 3,3-dimethoxy-4,4'-biphenyl diisocyanate, ethylene diamine, propane diamines, butanediamines, hexanediamines, pentane diamines, heptane diamines, octane diamines, m-xylylene diamine, 1,4-cyclohexane diamine, 2-methypentamethylene diamine, 4,4'-methylene dianiline, alkanol amines and diamines, ethylene glycol, diethylene glycol, triethylene glycol, 1,4-butanediol, neopentyl alcohol, 1,6-hexanediol, 1,8-octanediol, propylene glycols, 2,3-butylene glycol, dipropylene glycol, dibutylene glycol, glycerol, and mixtures and combinations thereof.

6. The device of any preceding claim , wherein the device is a vascular graft, a stent graft, or a vascular patch.

7. The device of any preceding claim comprising a reinforcing layer disposed on an outer most surface of the polymeric layer and non-porous silicone coating.

8. The device of claim 7, wherein the reinforcing layer comprises a plurality of stents.

9. The device of claim 8, wherein the stents comprise a material selected from a group consisting of stainless steel, nickel, silver, platinum, palladium, gold, titanium, tantalum, iridium, tungsten, a self-expanding nickel titanium alloy, nitinol, and Inconel.

10. A method of making a polyethylene terephthalate (PET) graft having a reduced permeability, comprising:
providing a porous graft having a first surface and a second surface, wherein the first surface and the second surface are positioned on opposite sides of the polymeric layer; and
applying a silicone composition comprising a two-part dispersion of silicone elastomer material dispersed in xylene to at least the first surface of the graft, said silicone composition permeating the graft so as to become embedded in the graft to produce a pore-free coating on the graft, the coating comprising silicone, ethyltriacetoxysilane and xylene, such that the graft or a portion thereof has a substantially reduced permeability or is entirely impermeable,
wherein the coating does not substantially increase the thickness of the graft, and
wherein the coating reduces the surface friction of the graft.

11. The method of claim 10, comprising at least one of: pretreating the graft comprising washing the graft in methylene chloride, acetone, or a mixture thereof; and curing the coated graft with an increased temperature in a range from about 30°C to about 150°C following the applying step.

12. A method for making a vascular prosthesis comprising
providing a porous graft layer comprising a porous PET fabric, the graft layer having a first surface and a second surface, wherein the first surface and the second surface are positioned on opposite sides of the polymeric layer; and
applying a silicone composition comprising a two-part dispersion of silicone elastomer material dispersed in xylene to at least the first surface of the graft, said silicone composition permeating the graft layer so as to become embedded in the graft to produce a first pore-free coating on the graft layer, the coating comprising silicone, ethyltriacetoxysilane, and xylene, such that the graft layer or a portion thereof has a substantially reduced permeability or is entirely impermeable,
wherein the coating does not substantially increase the thickness of the graft layer, and
wherein the coating reduces the surface friction of the graft layer.

13. The method of claim 12, comprising applying the silicone composition to the second surface.

14. The method of any of claims 12 and 13, wherein the step of applying the silicone composition comprises applying the composition by spraying the silicone composition on the first surface of the graft layer.

15. The method of any of claims 12 to 14, comprising curing the silicone material with an increased temperature in a range from 30°C to 150 °C.

## Patentansprüche

1. Implantierbare medizinische Vorrichtung, umfassend:
(i) eine Polymerschicht aus porösem Material mit einer ersten Oberfläche und einer zweiten Oberfläche, wobei die erste Oberfläche und die zweite Oberfläche an gegenüberliegenden Seiten der Polymerschicht angeordnet sind; und
(ii) eine nichtporöse Siliconbeschichtung, die in der Polymerschicht eingebettet ist und aus einer zweiteiligen Dispersion von in Xylen dispergiertem Silicon-Elastomermaterial, wobei die Beschichtung Silicon, Ethyltriacetoxysilan und Xylen umfasst, auf wenigstens der ersten Oberfläche der Polymerschicht angeordnet, um die Polymerschicht zu durchdringen, gebildet ist,
wobei die Polymerschicht oder ein Teil davon eine wesentlich verringerte Durchlässigkeit aufweist oder völlig undurchlässig ist,
wobei die eingebettete Beschichtung die Dicke der implantierbaren medizinischen Vorrichtung nicht wesentlich erhöht und
wobei die eingebettete Beschichtung die Oberflächenreibung der implantierbaren medizinischen Vorrichtung verringert.

2. Vorrichtung gemäß Anspruch 1, wobei die eingebettete nichtporöse Beschichtung Silicon, Ethyltriacetoxysilan und Xylen umfasst.

3. Vorrichtung gemäß einem der vorstehenden Ansprüche, wobei die Polymerschicht wenigstens eines von einem Polyethylenterephthalatgewebe; einem Polyethylen mit ultrahohem Molekulargewicht; und einem Polyurethanmaterial, das ein weiches Segment und ein hartes Segment umfasst, enthält.

4. Vorrichtung gemäß einem der Ansprüche 1 und 2, wobei die Polymerschicht ein Polyurethanmaterial umfasst, das ein weiches Segment und ein hartes Segment umfasst, wobei das weiche Segment eine oder mehrere Verbindungen ausgewählt aus der Gruppe bestehend aus Polyethylenoxid, Polypropylenoxid, Polytetramethylenoxid, Polycarbonat, Polyolefin, Polysiloxan, weichen Polyethersegmenten aus höheren homologen Reihen von Diolen, und Gemischen und Kombinationen davon umfasst.

5. Vorrichtung gemäß einem der Ansprüche 1 und 2, wobei die Polymerschicht ein Polyurethanmaterial umfasst, das ein weiches Segment und ein hartes Segment umfasst, wobei das harte Segment eine oder mehrere Verbindungen ausgewählt aus der Gruppe bestehend aus 4,4'-Diphenylmethandiisocyanat, Tetramethylendiisocyanat, Hexamethylendiisocyanat, Trimethyhexamethylendiisocyanat, Tetramethylxylylendiisocyanat, 4,4'-Decyclohexylmethandiisocyanat, Dimersäurediisocyanat, Isophorondiisocyanat, Metaxylendiisocyanat, Diethylbenzoldiisocyanat, Decamethylen-1,10-diisocyanat, Cyclohexylen-1,2-diisocyanat, 2,4-Toluoldiisocyanat, 2,6-Toluoldiisocyanat, Xylendiisocyanat, m-Phenylendiisocyanat, Hexahydrotolylendiisocyanat (und Isomere), Naphthylen-1,5-diisocyanat, 1-Methoxyphenyl-2,4-diisocyanat, 4,4'-Biphenylendiisocyanat, 3,3-Dimethoxy-4,4'-biphenyldiisocyanat, Ethylendiamin, Propandiaminen, Butandiaminen, Hexandiaminen, Pentandiaminen, Heptandiaminen, Octandiaminen, m-Xylylendiamin, 1,4-Cyclohexandiamin, 2-Methypentamethylendiamin, 4,4'-Methylendianilin, Alkanolaminen und -diaminen, Ethylenglycol, Diethylenglycol, Triethylenglycol, 1,4-Butandiol, Neopentylalkohol, 1,6-Hexandiol, 1,8-Octandiol, Propylenglycolen, 2,3-Butylenglycol, Dipropylenglycol, Dibutylenglycol, Glycerol und Gemischen und Kombinationen davon umfasst.

6. Vorrichtung gemäß einem der vorstehenden Ansprüche, wobei die Vorrichtung ein Gefäßtransplantat, ein Stenttransplantat oder ein Gefäß-Patch ist.

7. Vorrichtung gemäß einem der vorstehenden Ansprüche, umfassend eine Verstärkungsschicht, die auf einer am weitesten außen liegenden Oberfläche der Polymerschicht und der nichtporösen Siliconbeschichtung angeordnet ist.

8. Vorrichtung gemäß Anspruch 7, wobei die Verstärkungsschicht eine Vielzahl von Stents umfasst.

9. Vorrichtung gemäß Anspruch 8, wobei die Stents ein Material ausgewählt aus der Gruppe bestehend aus rostfreiem Stahl, Nickel, Silber, Platin, Palladium, Gold, Titan, Tantal, Iridium, Wolfram, einer selbstexpandierenden Nickel-Titan-Legierung, Nitinol und Inconel umfassen.

10. Verfahren zur Herstellung eines Polyethylenterephthalat(PET)-Transplantats mit einer verringerten Durchlässigkeit, umfassend:
Bereitstellen eines porösen Transplantats mit einer ersten Oberfläche und einer zweiten Oberfläche, wobei die erste Oberfläche und die zweite Oberfläche an gegenüberliegenden Seiten der Polymerschicht angeordnet sind; und
Aufbringen einer Siliconzusammensetzung, die eine zweiteilige Dispersion von in Xylen dispergiertem Silicon-Elastomermaterial umfasst, auf wenigstens die erste Oberfläche des Transplantats, wobei die Siliconzusammensetzung das Transplantat durchdringt, um in dem Transplantat eingebettet zu werden, um eine porenfreie Beschichtung auf dem Transplantat zu erzeugen, wobei die Beschichtung Silicon, Ethyltriacetoxysilan und Xylen umfasst, so dass das Transplantat oder ein Teil davon eine wesentlich verringerte Durchlässigkeit aufweist oder völlig undurchlässig ist,
wobei die Beschichtung die Dicke des Transplantats nicht wesentlich erhöht und
wobei die Beschichtung die Oberflächenreibung des Transplantats verringert.

11. Verfahren gemäß Anspruch 10, umfassend wenigstens eines von: Vorbehandeln des Transplantats, umfassend Waschen des Transplantats in Methylenchlorid, Aceton oder einem Gemisch davon; und Härten des beschichteten Transplantats bei einer erhöhten Temperatur in dem Bereich von etwa 30 °C bis etwa 150 °C nach dem Schritt des Aufbringens.

12. Verfahren zur Herstellung einer Gefäßprothese, umfassend
Bereitstellen einer porösen Transplantatschicht, die ein poröses PET-Gewebe umfasst, wobei die Transplantatschicht eine erste Oberfläche und eine zweite Oberfläche aufweist, wobei die erste Oberfläche und die zweite Oberfläche an gegenüberliegenden Seiten der Polymerschicht angeordnet sind; und
Aufbringen einer Siliconzusammensetzung, die eine zweiteilige Dispersion von in Xylen dispergiertem Silicon-Elastomermaterial umfasst, auf wenigstens die erste Oberfläche des Transplantats, wobei die Siliconzusammensetzung die Transplantatschicht durchdringt, um in dem Transplantat eingebettet zu werden, um eine erste porenfreie Beschichtung auf der Transplantatschicht zu erzeugen, wobei die Beschichtung Silicon, Ethyltriacetoxysilan und Xylen umfasst, so dass die Transplantatschicht oder ein Teil davon eine wesentlich verringerte Durchlässigkeit aufweist oder völlig undurchlässig ist,
wobei die Beschichtung die Dicke der Transplantatschicht nicht wesentlich erhöht und
wobei die Beschichtung die Oberflächenreibung der Transplantatschicht verringert.

13. Verfahren gemäß Anspruch 12, umfassend Aufbringen der Siliconzusammensetzung auf die zweite Oberfläche.

14. Verfahren gemäß einem der Ansprüche 12 und 13, wobei der Schritt des Aufbringens der Siliconzusammensetzung Aufbringen der Zusammensetzung durch Sprühen der Siliconzusammensetzung auf die erste Oberfläche der Transplantatschicht umfasst.

15. Verfahren gemäß einem der Ansprüche 12 bis 14, umfassend Härten des Siliconmaterials bei einer erhöhten Temperatur in dem Bereich von 30 °C bis 150 °C.

## Revendications

1. Dispositif médical implantable comprenant :
(i) une couche polymère de matériau poreux ayant une première surface et une deuxième surface, la première surface et la deuxième surface étant positionnées sur des côtés opposés de la couche polymère ; et
(ii) un revêtement de silicone non poreux incorporé à l'intérieur de la couche polymère et formé à partir d'une dispersion en deux parties de matériau élastomère de silicone dispersé dans du xylène, le revêtement comprenant de la silicone, de l'éthyltriacétoxysilane et du xylène, disposés au moins sur la première surface de la couche polymère pour perméer à travers la couche polymère,
dans lequel la couche polymère ou une partie de celle-ci a une perméabilité sensiblement réduite ou est totalement imperméable,
dans lequel le revêtement incorporé n'augmente pas sensiblement l'épaisseur du dispositif médical implantable, et
dans lequel le revêtement incorporé réduit le frottement de surface du dispositif médical implantable.

2. Dispositif de la revendication 1, dans lequel le revêtement non poreux incorporé comprend de la silicone, de l'éthyltriacétoxysilane et du xylène.

3. Dispositif d'une quelconque revendication précédente, dans lequel la couche polymère comporte au moins un élément parmi un tissu de téréphtalate de polyéthylène ; un polyéthylène à ultrahaut poids moléculaire ; et un matériau de polyuréthane comprenant un segment mou et un segment dur.

4. Dispositif de l'une quelconque des revendications 1 et 2, dans lequel la couche polymère comprend un matériau de polyuréthane comprenant un segment mou et un segment dur, le segment mou comprenant un ou plusieurs composés choisis dans le groupe constitué par l'oxyde de polyéthylène, l'oxyde de polypropylène, l'oxyde de polytétraméthylène, le polycarbonate, une polyoléfine, le polysiloxane, des segments mous de polyéther fabriqués à partir de séries homologues supérieures de diols, et les mélanges et combinaisons de ceux-ci.

5. Dispositif de l'une quelconque des revendications 1 et 2, dans lequel la couche polymère comprend un matériau de polyuréthane comprenant un segment mou et un segment dur, le segment dur comprenant un ou plusieurs composés choisis dans le groupe constitué par le diisocyanate de 4,4'-diphénylméthane, le diisocyanate de tétraméthylène, le diisocyanate d'hexaméthylène, le diisocyanate de triméthylhexaméthylène, le diisocyanate de tétraméthylxylylène, le diisocyanate de 4,4'-dicyclohexylméthane, un diisocyanate d'acide dimère, le diisocyanate d'isophorone, le diisocyanate de métaxylène, le diisocyanate de diéthylbenzène, le 1,10-diisocyanate de décaméthylène, le 1,2-diisocyanate de cyclohexylène, le diisocyanate de 2,4-toluène, le diisocyanate de 2,6-toluène, le diisocyanate de xylène, le diisocyanate de m-phénylène, le diisocyanate d'hexahydrotolylène (et ses isomères), le 1,5-diisocyanate de naphtylène, le 2,4-diisocyanate de 1-méthoxyphényle, le diisocyanate de 4,4'-biphénylène, le diisocyanate de 3,3-diméthoxy-4,4'-biphényle, l'éthylènediamine, les propanediamines, les butanediamines, les hexanediamines, les pentanediamines, les heptanediamines, les octanediamines, la m-xylylènediamine, la 1,4-cyclohexanediamine, la 2-méthylpentaméthylènediamine, la 4,4'-méthylènedianiline, les alcanolamines et alcanoldiamines, l'éthylène glycol, le diéthylène glycol, le triéthylène glycol, le 1,4-butanediol, l'alcool néopentylique, le 1,6-hexanediol, le 1,8-octanediol, les propylène glycols, le 2,3-butylène glycol, le dipropylène glycol, le dibutylène glycol, le glycérol, et les mélanges et combinaisons de ceux-ci.

6. Dispositif d'une quelconque revendication précédente, le dispositif étant un greffon vasculaire, un greffon de stent, ou une pièce vasculaire.

7. Dispositif d'une quelconque revendication précédente comprenant une couche de renforcement disposée sur une surface la plus à l'extérieur de la couche polymère et du revêtement de silicone non poreux.

8. Dispositif de la revendication 7, dans lequel la couche de renforcement comprend une pluralité de stents.

9. Dispositif de la revendication 8, dans lequel les stents comprennent un matériau choisi dans un groupe constitué par l'acier inoxydable, le nickel, l'argent, le platine, le palladium, l'or, le titane, le tantale, l'iridium, le tungstène, un alliage nickel-titane auto-expansible, le nitinol, et l'Inconel.

10. Procédé de fabrication d'un greffon de téréphtalate de polyéthylène (PET) ayant une perméabilité réduite, comprenant :
l'obtention d'un greffon poreux ayant une première surface et une deuxième surface, la première surface et la deuxième surface étant positionnées sur des côtés opposés de la couche polymère ; et
l'application d'une composition de silicone comprenant une dispersion en deux parties de matériau élastomère de silicone dispersé dans du xylène au moins à la première surface du greffon, ladite composition de silicone perméant à travers le greffon de manière à être incorporée dans le greffon pour produire un revêtement sans pores sur le greffon, le revêtement comprenant de la silicone, de l'éthyltriacétoxysilane et du xylène, de telle sorte que le greffon ou une partie de celui-ci a une perméabilité sensiblement réduite ou est totalement imperméable,
dans lequel le revêtement n'augmente pas sensiblement l'épaisseur du greffon, et
dans lequel le revêtement réduit le frottement de surface du greffon.

11. Procédé de la revendication 10, comprenant au moins une des opérations suivantes : prétraitement du greffon comprenant un lavage du greffon dans du chlorure de méthylène, de l'acétone, ou un mélange de ceux-ci ; et durcissement du greffon revêtu avec une température accrue dans une gamme d'environ 30 °C à environ 150 °C après l'étape d'application.

12. Procédé de fabrication d'une prothèse vasculaire comprenant
l'obtention d'une couche de greffon poreux comprenant un tissu de PET poreux, la couche de greffon ayant une première surface et une deuxième surface, la première surface et la deuxième surface étant positionnées sur des côtés opposés de la couche polymère ; et
l'application d'une composition de silicone comprenant une dispersion en deux parties de matériau élastomère de silicone dispersé dans du xylène au moins à la première surface du greffon, ladite composition de silicone perméant à travers la couche de greffon de manière à être incorporée dans le greffon pour produire un premier revêtement sans pores sur la couche de greffon, le revêtement comprenant de la silicone, de l'éthyltriacétoxysilane et du xylène, de telle sorte que la couche de greffon ou une partie de celle-ci a une perméabilité sensiblement réduite ou est totalement imperméable,
dans lequel le revêtement n'augmente pas sensiblement l'épaisseur de la couche de greffon, et
dans lequel le revêtement réduit le frottement de surface de la couche de greffon.

13. Procédé de la revendication 12, comprenant l'application de la composition de silicone à la deuxième surface.

14. Procédé de l'une quelconque des revendications 12 et 13, dans lequel l'étape d'application de la composition de silicone comprend l'application de la composition par pulvérisation de la composition de silicone sur la première surface de la couche de greffon.

15. Procédé de l'une quelconque des revendications 12 à 14, comprenant le durcissement du matériau de silicone avec une température accrue dans une gamme de 30 °C à 150 °C.
